# EUROPÄISCHE PATENTSCHRIFT

(11) **EP 1 026 531 B1**
(45) Veröffentlichungstag und Bekanntmachung des Hinweises auf die Patenterteilung: **22.03.2006**
(21) Anmeldenummer: 00101142.8
(22) Anmeldetag: 21.01.2000
(51) Int. Cl.: G02B 7/00

(54) **Stativ, insbesondere Mikroskopstativ**
Support intended in particular for a surgical microscope
Pied en particulier pour microscope d'opérations

(30) Priorität: 07.02.1999 CH 26699
(43) Veröffentlichungstag der Anmeldung: 09.08.2000
(73) Patentinhaber: Leica Microsystems AG, 9435 Heerbrugg (CH)
(72) Erfinder: Metelski, Andreas, 8590 Romanshorn (CH)
(74) Vertreter: Reichert, Werner Franz

(56) Entgegenhaltungen:
- EP-A- 0 023 004
- WO-A-97/20166
- WO-A-98/53244
- US-A- 3 119 588
- US-A- 3 637 233

## Beschreibung

Die Erfindung betrifft ein Stativ nach Anspruch 1, insbesondere ein Mikroskopstativ, wie es z.B. für Operationsstative zum Einsatz gelangt und ein Verfahren zur Montage eines Stativfusses nach Anspruch 13.

Eines dieser Stative mit der Bezeichnung "S5" von Zeiss verfügt über einen hohlen Stativfusskörper, in den von der Unterseite Gewichte eingeschoben werden können, die mittels querliegenden Blechstreifen und Schrauben im Hohlraum des Stativfusskörpers fixiert bzw. verriegelt werden können. Die Gewichte dienen der verbesserten Kippsicherheit des Statives.

Der Nachteil dieses bekannten Aufbaus ist das hohe Gewicht, das sich nach Einbau der Gewichte ergibt. Dadurch, dass die Gewichte von der Unterseite her eingebaut werden müssen, wird der Stativfuss bei der Montage auf seine Oberseite gelegt. Nach dem Einbau der Gewichte, muss der nun beschwerte Stativfuss wieder gewendet werden. Dies ist nur unter grosser Kraftanstrengung bzw. unter Zuhilfenahme mehrerer Personen möglich. Der Effekt, der somit durch die Aufteilung in mehrere Gewichte erzielt wird, wird durch diese Art des Einbaus zum Teil wieder zunichte gemacht. Dadurch werden auch zulässige Arbeitsnormen für eine Person überschritten.

Ein weiteres Mikroskopstativ mit gewichtsausgleich ist aus WO 99/01693 A bekannt.

Der Erfindung liegt daher die Aufgabe zugrunde, ein verbessertes System und Montageverfahren zu schaffen, bei dem im Zuge der Monatge nie das Gesamtgewicht des Stativfusses gehoben, bzw. bei dem der Stativfuss im montierten Zustand grundsätzlich nicht gewendet werden muss.

Die Kombination der Merkmale des Anspruches 1 löst diese Hauptaufgabe.

Die neue Bedienbarkeit der Fixiervorrichtung für das Gewicht von einer anderen Seite als der Unterseite des Stativfusses erspart den oben beschriebenen Wendevorgang.

Dies ist nicht nur bei der Erstmontage sondern auch bei jeder weiteren Montage, z.B. zu Transportzwecken, Servicezwecken und Demonstrationen o.dgl. ein Vorteil. Noch weiter verbesserte Lösungen mit weitergehender Integration und weitergehenden Vorteilen gegenüber dem Stand der Technik ergeben sich aus den abhängigen Ansprüchen.

Weitere Verbesserungen und erfindungsgemässe Details ergeben sich aus der Zeichnung, die ein erfindungsgemässes Ausführungsbeispiel darstellt. Die Figuren werden übergreifend beschrieben. Gleiche Bezugszeichen bedeuten dabei gleiche Bauteile. Funktionsgleiche Bauteile mit eventuell unterschiedlicher Formgebung tragen gleiche Bezugszeichen mit unterschiedlichen Indizes. Die Bezugszeichenliste ist integrierender Bestandteil der Figurenbeschreibung. Die beschreibenden Elemente der Patentansprüche gelten als im Rahmen der obigen Beschreibungseinleitung und/oder der nachfolgenden Figurenbeschreibung inkorporiert.

Es zeigen dabei:
- Fig.1: eine Ansicht auf ein erfindungsgemässes Stativ in schematischer Darstellung vor dem Zusammenbau;
- Fig.2: eine Variante zum Aufbau der Fig.1;
- Fig.3: eine weitere Variante zum Aufbau der Fig.1 in Draufsicht;
- Fig.4: den Aufbau von Fig.3 in Schrägansicht;
- Fig.5: eine ausgeführte Variante zum Aufbau nach Fig.1 in einem Seitenschnitt durch den Stativfusskörper;
- Fig.6: die Draufsicht auf den Aufbau nach Fig.5;
- Fig.7: ein Detail einer Variante zum Aufbau nach Fig.5;
- Fig.8: eine Detailvariante zu Fig.5 und
- Fig.9: einen Schnitt durch die Wandung eines Stativfusskörpers nach Fig.8 zwischen benachbarten, sternförmigen Armen.

Die Figuren sind übergreifend beschrieben. Gleiche Bezugszeichen bedeuten gleiche Bauteile. Gleiche Bezugszeichen mit unterschiedlichen Indizes bedeuten Bauteile mit gleichen Aufgaben aber geringfügig anderem Aufbau. Die Figuren schränken die Erfindung nicht ein, sondern zeigen lediglich Ausführungsbeispiele der Erfindung.

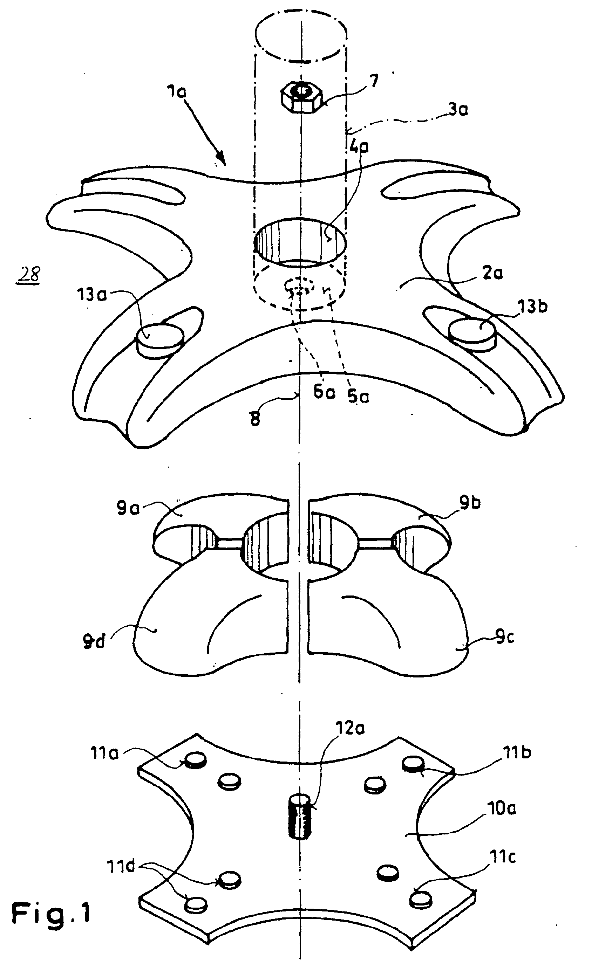

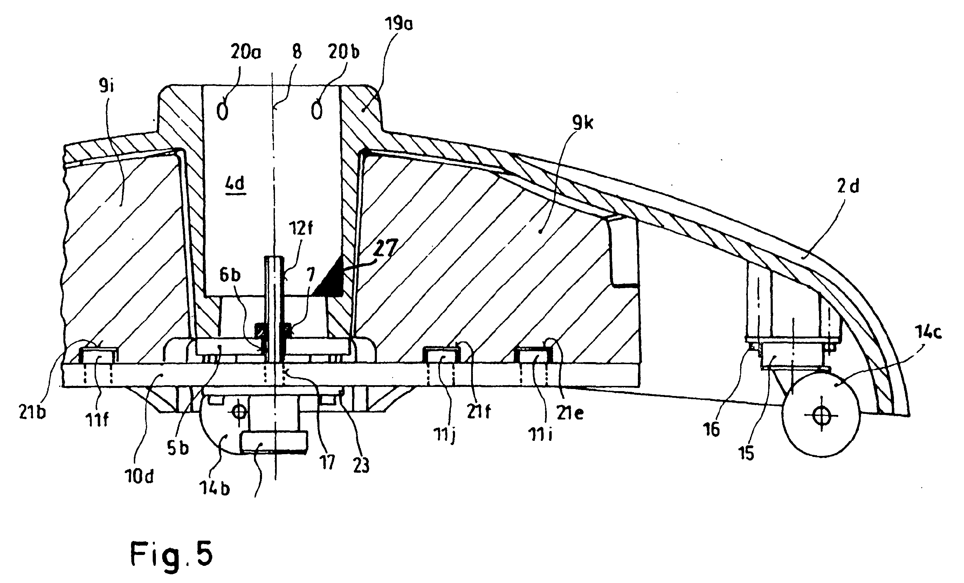

Aus Fig.1 ist eine der bevorzugten Ausführungsformen der Erfindung zu erkennen. Auf einer in Gebrauchslage am Boden liegende Montagehalterung 10a sind vier einzelne Gewichte 9a-d mittels Rasten 11a-d und gegengleichen Rastausnehmungen (vgl. 21a-h in Fig.5) in den Gewichten 9a-d vorpositioniert. Die Feinpositionierung und Verriegelung der Gewichte 9a-d erfolgt durch das Aufsetzen eines Stativfusskörpers 2a, der über entsprechende Hohlräume zur Aufnahme der Gewichte 9a-d verfügt. Der Stativfusskörper 2a verfügt in der Mitte über eine Ausnehmung 4a, deren Boden 5a mit einer Bohrung 6a durchsetzt ist. Die Bohrung 6a passt auf eine Gewindestange 12a, die mit der Grundplatte 10a fest verbunden - zB. verschraubt, verpresst oder verschweisst - ist. Auf diese Gewindestange 12a wird zum Verriegeln eine Mutter 7 gedreht, so lange, bis sich die Montagehalterung 10a über die Gewichte 9a-d am Stativfusskörper abstützt. Dabei feinpositionieren die den Hohlraum oder die Hohlräume des Stativfusskörpers begrenzenden Wandungen die Gewichte 9a-d. Insbesondere sind es konisch ausgebildete Wandungen, die diese Feinpositionierung bewerkstelligen.

Der Stativfuss 1a dient dabei auch zur Aufnahme eines Ständers 3a, der in der Ausnehmung 4a verankert werden kann. Der Stativfuss 1a trägt dabei ausser Transportrollen 14 (in Fig.1 nicht dargestellt) auch zwei Bremsen, von denen in Fig.1 die Bremspedale 13a,b angedeutet sind.

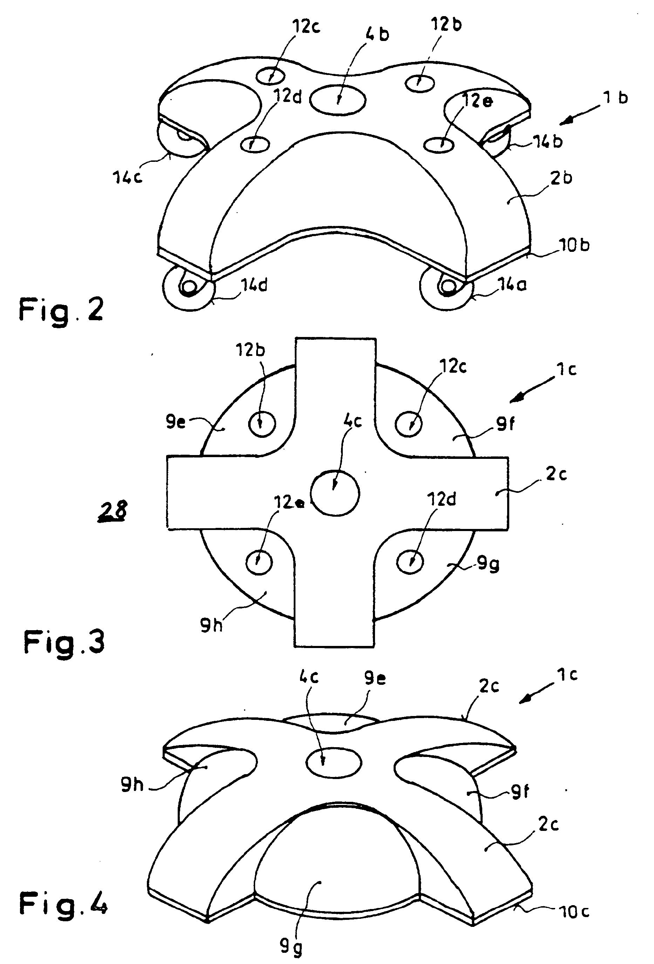

Die Variante gemäss Fig.2 verfügt ebenso über einen Stativfusskörper 2b, der aus vier armförmigen Stativfusskörperteilen aufgebaut ist. Jeder Teil verfügt über einen Hohlraum zur Aufnahme eines Gewichtes. Jeder Teil wird zusammen mit dem im Hohlraum eingeschlossenen Gewicht mittels Gewindestange 12b-e gleich oder ähnlich dem Aufbau gemäss Fig.1 gegenüber einer Montagehalterung 10b von oben her verschraubt.

Als Variante dazu könnten die Schrauben auch seitlich die Gewichte 9e-h durchsetzen und in Montagelage annähernd waagrecht zum Boden 28 liegen. Dies ermöglicht immer noch den Zugang vom Raum her, reduziert andererseits aber Schmutzangriffsflächen an der Oberseite der Gewichte.

Auch hier wird somit der erfindungsgemäss angestrebte Effekt erzielt und eine Montage kann von oben erfolgen. Selbstverständlich könnte der Aufbau des Stativfusskörpers 2b auch einstückig sein. Die Darstellung der Fig.2 dient dem Hinweis, dass erfindungsgemäss auch mehrere Schrauben zur Verbindung des Stativfusskörpers mit der Montagehalterung angewendet werden können.

Ausserdem sei erwähnt, dass im Rahmen der Erfindung auch Varianten liegen, bei denen eine Schraube von oben nach unten in die Montagehalterung durch den Stativfusskörper und gegebenenfalls durch das jeweilige Gewicht gedreht werden kann. Auch andere Verriegelungsarten, wie etwa seitlich aufgeschobene Klammern o.dgl. sind umfasst, jedoch sind die dargestellten Varianten bevorzugt.

Fig.2 dient auch als Hinweis auf die erfindungsgemäss vorgesehene Möglichkeit, Transportrollen 14a-d mit der Montagehalterung 10b zu verbinden, so dass im Unterschied zur Fig.5 z.B. die Rollen 14a-d nicht unmittelbar mit dem Stativfusskörper 2b verbunden sind.

Fig.3 zeigt eine weitere Variante, bei der die Gewichte nicht durch einen Hohlraum aufgenommen sondern seitlich an den Stativfusskörper 2c angeschraubt werden. Diese Variante ist aus Gründen der Sterilisierung des Operationsmikroskop-Stativs nicht bevorzugt, da zwischen den Gewichten 9e-h Spalten verbleiben. Für weniger kritische Bereiche ist dieser einfache Aufbau jedoch mit Vorteil einsetzbar. Die nur symbolisch angedeuteten Gewindestangen 12b-e oder Schrauben können bei diesem Aufbau auch schräg in den Stativfusskörper 2c eingedreht werden, bzw. aus diesem schräg nach aussen abragen.

Fig.4 zeigt den Aufbau von Fig.3 in Schrägansicht

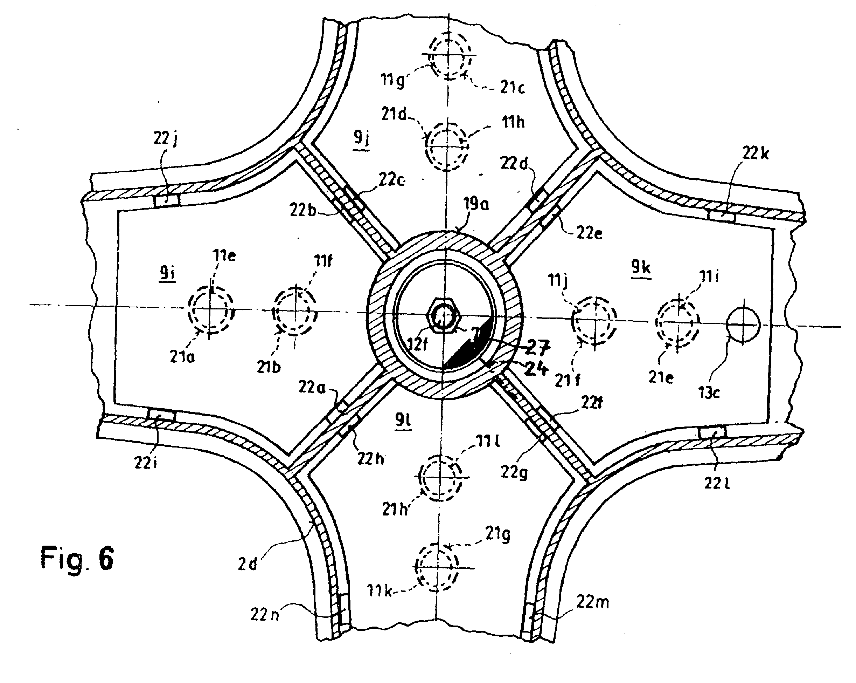

Der Aufbau des Stativfusses gemäss den Fig.5 und 6 ist eine bevorzugte Ausführungsform.

Zusätzlich zum schon Beschriebenen sieht man eine besondere Formgebung der Gewichte mit Rastausnehmungen 21a-h. Die Rastausnehmungen können selbstverständlich auch unterschiedlich geformt sein, um gegebenenfalls einen grösseren Freiheitsgrad beim Feinpositionieren zu haben. Den Rastausnehmungen 21a-h sind Rasten 11e-l gegenübergestellt, die nietförmig aussehen und bevorzugt aus Kunststoff gefertigt sind. Sie sind in Bohrungen der Montagehalterung 10d verankert. Der Vorteil solcher Kunststoffrasten liegt in der geringeren Abnützungsgefahr für die Gewichte, die z.B. nicht aus rostfreiem Material gefertigt sind. Zwar werden solche Gewichte gemäss einer besonderen Ausgestaltung bevorzugt grundiert oder lackiert, jedoch sind gerade an Raststellen Abnützungen möglich, die das rostende Material freilegen könnten. Dies wird wirkungsvoll durch den Kunststoff verhindert.

Abgesehen davon können die Rasten und Rastausnehmungen auch so ausgebildet sein, dass im Montagezustand die Unterseite der Gewichte 9i-l von der Oberseite der Montagehalterung 10d beabstandet ist, so dass die Gewichte 9i-l auf den Rasten 11e-l ruhen.

Alternativ dazu könnten auch Schaumstoffstreifen zwischen die Unterseite der Gewichte und Oberseite der Montagehalterung eingebracht sein, so dass die Gewichte an allen Seiten gegen mechanische Abnützung geschützt sind. Dabei sind sie dann auch gleichzeitig schwingungsgedämpft.

Die Transportrollen 14b,c sind herkömmliche Rollen.

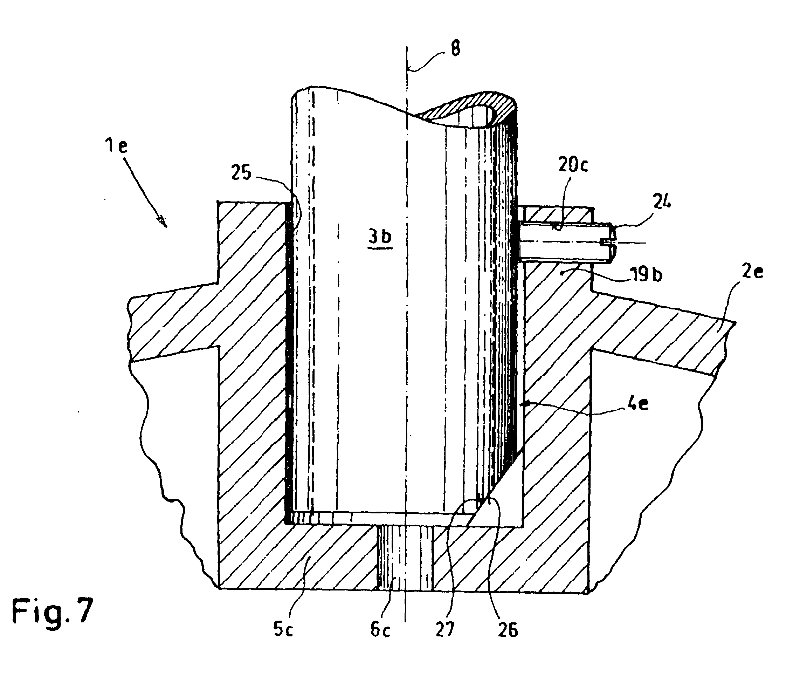

Die Ausnehmung 4d verfügt im Bereich ihrer Stützwand 19a über mindestens eine Gewindebohrung 20a,b, die mit einer nicht gezeigten Schraube der Arretierung des nicht gezeigten Ständers 3a (Fig.5,7) dient.

Als Besonderheit verfügt der Aufbau gemäss Fig.5 und 6 über eine eingeschraubte Gewindestange 12f, die an ihrer der Montagehalterung 10d zugewandten Seite einen Imbus aufweist, über den sie in die Montagehalterung 10d geschraubt werden kann. Zur Sicherheit kann dieser Bereich mittels Deckplatte 23 abgedeckt sein.

In Fig.6 sind beispielhaft Schaumstoffstreifen 22a-m dargestellt, die an den, die jeweiligen Hohlräume begrenzenden Wandungen des Stativfusskörpers 2d angebracht sind und im montierten Zustand den Gewichten 9i-1 einen wackelsicheren und gedämpften Sitz in den Hohlräumen gewährleisten. Sie dienen so auch dem Spielausgleich.

Ausserdem erkennt man angedeutet eine bevorzugt angewendete Schrägfläche bzw. einen Keil 27 und eine symbolisch angedeutete Schraube 24, die in Fig.7 näher beschrieben sind.

Das besondere Detail gemäss Fig.7 zeigt einen Ausschnitt eines Stativfusskörpers 2e, der in seiner Ausnehmung 4e im Bereich ihres Bodens 5c einen Keil 26 aufweist. Dieser Keil 26 bewirkt über seine Schrägfläche 27 eine seitliche Schiebekraft auf den eingesetzten Ständer 3b, so dass dieser mit der Schiebekraft zwangsläufig an die dem Keil gegenüberliegende Wandung 25 der Ausnehmung 4e gedrückt wird. Da diese Wandung in Betriebsstellung senkrecht zum Boden ist, wird die Säule somit ebenso senkrecht orientiert; und dies unabhängig von den Durchmessertoleranzen. Daraus ergibt sich eine Quasizentrierung. Das Zentrum des Ständers kann geringfügig aus dem Zentrum des Stativfusses verschoben sein (im Toleranzbereich der Ausnehmung 4e und des Ständers 3b). Der Ständer kann ebenso über eine Anschrägung im Bereich von 27 aufweisen, dies ist jedoch nicht zwingend.

Der Vorteil dieses Aufbaus, der auch unabhängig von dem anderen erfindungsgemässen Stativfussaufbaus eingesetzt werden kann, liegt in der Einfachheit der Befestigung des Ständers mit nur einer Klemmschraube 24 bei trotzdem sichergestellter "Zentrierung".

Gemäss einer besonderen Ausgestaltung der Erfindung ist die Ausnehmung 4 im Stativfusskörper 2 gegen Verschleiss geschützt. Dies kann z.B. mittels Gleitbüchse aus Stahl in der Ausnehmung des Alu-Stativfusskörpers bewerkstelligt sein.

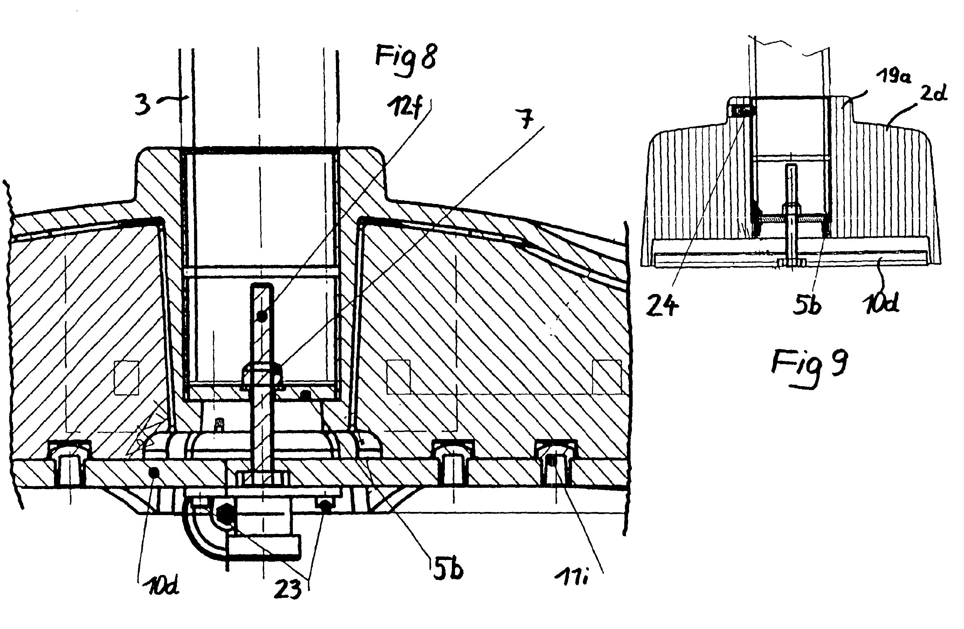

Die Platte 5b gemäss Fig.5 kann zu Montagezwecken auch mittels nicht dargestellter Schrauben am Stativfusskörper 2d befestigt sein. Eine Variante dazu ist in Fig.8 dargestellt. Die Platte 5b ist hier nicht von unten sondern von oben auf einen Vorsprung der Wandung des Stativfusskörpers aufgelegt. Die Schraube 12f ist an ihrem unteren Ende mit einer Mutter an der Montagehalterung 10d gehalten. Eine Platte 29 sichert Die Rasten 11i sind bei diesem Beispiel pilzförmig und aus Kunststoff aufgebaut.

In allen Figuren ist die Montagehalterung als Bodenplatte dargestellt. Die Erfindung umfasst jedoch auch Varianten, bei denen einen Bodenplatte fehlt und die Gewichte an einer im Schnitt bügelförmigen Montagehalterung befestigbar sind, im montierten Zustand somit nicht auf der Montagehalterung stehen, sondern an ihr hängen.

Bei Alternativen dazu fehlt neben der Bodenplatte auch eine bügelförmige Montagehalterung. Bei solchen Alternativen sind Stützabsätze am Stativfusskörper o.dgl. für das bzw. die Gewichte vorgesehen, wobei das bzw. die Gewichte mit dem Fusskörper verschraubt werden. Solche Stützabsätze kann man sich z.B. in Fig.4 anstelle der Bodenplatte 10c vorstellen. Sie wären z.B. mit dem Fusskörper 2c an seinem unteren Rand verbunden und würden so ebenso wie im gezeichneten Ausführungsbeispiel die Gewichte 9e-h stützen.

### Bezugszeichenliste

- 1: Stativfuss a,b,c,d,e
- 2: Stativfusskörper a,b,c,d,e
- 3: Ständer a,b
- 4: Ausnehmung a,b,c,d,e
- 5: Boden der Ausnehmung a,b,c (kann eine Platte sein, oder einstückig mit dem Stativfusskörper ausgebildet sein)
- 6: Bohrung a,b,c
- 7: Mutter
- 8: Achse des Ständers
- 9: Gewicht(e) a-d,e-h,i-l
- 10: Montagehalterung a,b,c,d
- 11: Rasten a-d,e-l
- 12: Gewindestange a,b-e,f
- 13: Bremspedal a-b, c
- 14: Transportrollen a-d
- 15: Rollensockel
- 16: Schraube
- 17: Pressitz oder Gewindesitz
- 18: Bremsklotz
- 19: Stützwand a,b
- 20: Gewindebohrung a,b,c
- 21: Rastausnehmung a-h
- 22: Schaumstoffstreifen a-m
- 23: Deckplatte
- 24: Klemmschraube
- 25: Wandung
- 26: Keil
- 27: Schrägfläche
- 28: Boden

## Patentansprüche

1. Mikroskopstativ mit einem Ständer (3) und einem Stativfuss (1) zum Aufstellen auf einem Boden (28) in einem Raum, mit wenigstens einem Stativfusskörper (2), der mittels wenigstens einem Gewicht (9) bestückbar ausgebildet ist, mit einer Fixiervorrichtung (5,6,7,10,12) zum Fixieren des Gewichts (9) am Stativfusskörper (2), **dadurch gekennzeichnet, dass** die Fixiervorrichtung (5,6,7,10,12) eine Verriegelungsvorrichtung (5,6,7,12) und eine Montagehalterung (10)zur Aufnahme von wenigstens einem der Gewichte (9) umfassen, wobei die Verriegelungsvorrichtung (5,6,7,12) von der dem Ständer (3) zugewandten Seite des Stativfusskörpers (2a;2b;2d;2e) betätigbar ist und dabei die Montagehalterung (10), zusammen mit dem wenigstens einem Gewicht (9), mit dem Stativfusskörper (2a;2b;2d;2e) von der dem Ständer (3) abgewandten Seite des Stativfusskörpers (2a;2b;2d;2e) verbindet.

2. Mikroskopstativ nach Anspruch 1, **dadurch gekennzeichnet, dass** das wenigstens ein Gewicht (9a-d;9i-l) an der Montagehalterung (10a;10d) grobpositionierbar ist, **und dass** der Stativfusskörper einen hohlen Körper mit wenigstens einem Hohlraum aufweist, wobei wenigstens zwei einen bzw. den wenigstens einen Hohlraum umschliessende Wandungen so ausgebildet sind, dass sie während des Verriegelungsvorgangs das Gewicht (9a-d;9i-l) feinpositionieren.

3. Mikroskopstativ nach Anspruch 2, **dadurch gekennzeichnet, dass** wenigstens einer der beiden Wandungen an ihrer dem Gewicht (9a-d;9i-l) und in Betriebslage dem Boden (28) zugewandten Seite sich vom Boden weg konisch verbreiternd ausgebildet ist, **und/oder dass** wenigstens eine der Wandungen und/oder die Montagehalterung (10a;10b;10d) mittels einer elastischen Lage (22a-m), vorzugsweise aus einem Schaumstoff, beschichtet ist.

4. Mikroskopstativ nach einem der Ansprüche 1-3, **dadurch gekennzeichnet, dass** die Verriegelungsvorrichtung vorzugsweise wenigstens eine Muttern/Schrauben-Verbindung (7,22a;12f) umfasst, die den Stativfusskörper durchsetzt (2a;2b;2d) und diesen mit der Montagehalterung (10a;10b;10d) verschraubbar macht.

5. Mikroskopstativ nach Anspruch 4, **dadurch gekennzeichnet, dass** mehrere Gewichte (9a-d;9i-l) vorgesehen sind, **und dass** die Muttern/Schrauben-Verbindung (7,12a;12f) im Zentrum des Stativfusskörpers (2a;2b;2d), somit auch zentral in bezug auf die Gewichte (9a-d;9i-l) angeordnet ist.

6. Mikroskopstativ nach Anspruch 4 oder 5, **dadurch gekennzeichnet, dass** die Muttern/Schrauben-Verbindung (7,12a;12f) selbsthemmend ausgebildet **oder** mit einer Drehsicherung versehen ist.

7. Mikroskopstativ nach einem der vorhergehenden Ansprüche, **dadurch gekennzeichnet, dass** der Stativfusskörper (2a;2b;2d) mit sternförmigen Stützarmen ausgebildet ist, **und dass** jeder Arm einen Hohlraum oder einen Stützabsatz für je mindestens ein Gewicht (9a-d; 9i-l) und vorzugsweise je eine Abstützrolle (14) aufweist.

8. Mikroskopstativ nach einem der vorhergehenden Ansprüche, bei dem sowohl die Montageplatte (10), als auch der Stativfusskörper (2), als auch jedes Gewicht (9) jeweils ein im Rahmen zulässiger Arbeitsnormen bei Handhabung durch eine Person definiertes, maximales Gewicht von z.B. je ca. 10-25kg aufweisen.

9. Mikroskopstativ nach einem der vorhergehenden Ansprüche, **dadurch gekennzeichnet, dass** das bzw. die Gewicht(e) aus grundiertem und/oder lackiertem Grauguss, Stahl, Messing oder Schwermetall gefertigt sind, **und/oder dass** der Stativfusskörper aus Alu, insbesondere Aluminiumdruckguss hergestellt ist.

10. Mikroskopstativ nach einem der Ansprüche 1-9, **dadurch gekennzeichnet, dass** das Gewicht bzw. die Gewichte (9a-d;9i-l) über Rastausnehmungen (21) verfügen, die mit gegengleichen Rasten (11) der Montagehalterung (10a;10d) kooperieren, wobei die Rasten (11) vorzugsweise aus Kunststoff gefertigt sind, **und dass** vorzugsweise eine der Rastausnehmungen zylindrisch und die andere langlochförmig ausgebildet ist.

11. Mikroskopstativ nach einem der vorhergehenden Ansprüche, **dadurch gekennzeichnet, dass** das bzw. die Gewicht(e) (9) gegenüber dem Stativfusskörper (2) und/oder gegenüber der Montagehalterung (10) schwingungsgedämpft gelagert ist bzw. sind.

12. Mikroskopstativ nach Anspruch 1-11, **dadurch gekennzeichnet, dass** der Stativfusskörper (2d;2e) um die Achse (8) des Ständers (3) rotationssymmetrisch ausgebildet ist und in seinem Zentrum um die Achse (8) des Ständers eine rotationssymmetrische Ausnehmung (4) für den Ständer (3) aufweist, wobei die Ausnehmung (4) an ihrem, dem Boden (28) zugewandten Ende abweichend von der Rotationssymmetrie einen keilförmigen Vorsprung bzw. einen Keil (26) aufweist, der ein eingesetztes Ständerrohr (3) - vorzugsweise über eine dort ausgebildete gegengleiche Keilfläche (27) - durch dessen Eigengewicht zwingend an die gegenüberliegende Wand (25) der Ausnehmung (4) andrückt, wobei der Ständer (3) mit wenigstens einer Schraube (24) durch eine Stützwandung (19) der Ausnehmung (4) gegen die gegenüberliegende Wand (25) formschlüssig klemmbar ist.

13. Verfahren zum Montieren eines Mikroskopstativs, **dadurch gekennzeichnet, dass** eine Montagehalterung (10a;10d) bereitgestellt wird, dass auf dieser Montagehalterung (10a;10d) wenigstens ein Gewicht (9a-d;9i-l) grobpositioniert wird, dass an der Montagehalterung (10a;10d) etwa in ihrem Zentrum ein mit ihr fest verbundener Gewindezapfen (12a;12f) bereitgestellt wird, dass ein hohler Stativfusskörper (2a;2d) über die Montagehalterung (10a;10d) und auf das Gewicht (9a-d;9i-1) gestülpt wird, wobei der Gewindezapfen (12a;12f) eine Bohrung (6) des Stativfusskörpers (2a;2d) durchsetzt und wenigstens zwei den Hohlraum des Stativfusskörpers (2a;2d) begrenzende Wandungen das Gewicht (9a-d;9i-l) umfassen, worauf auf den Gewindezapfen (12a;12f) eine Mutter (7) aufgeschraubt wird, solange bis die Montagehalterung (10a;10d) fest mit dem Stativfusskörper (2a;2d) verbunden ist, wobei gleichzeitig die beiden Wandungen das Gewicht (9a-d;9i-l) auf der Montagehalterung (10a;10d) feinpositionieren.

## Claims

1. Microscope stand having a column (3) and a stand foot (1) for putting up on a floor (28) in a room, having at least one stand foot element (2) which is designed to be capable of being fitted with at least one weight (9), and having a fixing apparatus (5, 6, 7, 10, 12) for fixing the weight (9) on the stand foot element (2), **characterized in that** the fixing apparatus (5, 6, 7, 10, 12) comprises a locking apparatus (5, 6, 7, 12) and a mounting holder (10) for holding at least one of the weights (9), the locking apparatus (5, 6, 7, 12) can be operated from the side of the stand foot element (2a; 2b; 2d; 2e) facing the column (3) and in this case connects the mounting holder (10), together with the at least one weight (9), to the stand foot element (2a; 2b; 2d; 2e) from the side of the stand foot element (2a; 2b; 2d; 2e) that is averted from the column (3).

2. Microscope stand according to Claim 1, **characterized in that** the at least one weight (9a-d; 9i-l) can be coarsely positioned on the mounting holder (10a; 10d), and **in that** the stand foot element has a hollow body with at least one cavity, at least two walls surrounding one or the at least one cavity being designed such that they can be finely positioned during the locking operation of the weight (9a-d; 9i-l).

3. Microscope stand according to Claim 2, **characterized in that** at least one of the two walls is designed in a fashion widening conically away from the floor at its end facing the weight (9a-d; 9i-l) and, in the operating position of the floor (28), and/or **in that** at least one of the walls and/or the mounting holder (10a; 10b; 10d) is coated by means of an elastic layer (22a-m), preferably made from a foamed plastic.

4. Microscope stand according to one of Claims 1-3, **characterized in that** the locking apparatus preferably comprises at least one nut/screw connection (7, 12a; 12f) which penetrates the stand foot element (2a; 2b; 2d) and renders the latter capable of being screwed to the mounting holder (10a; 10b; 10d).

5. Microscope stand according to Claim 4, **characterized in that** a number of weights (9a-d; 9i-l) are provided and **in that** the nut/screw connection (7, 12a; 12f) is arranged at the centre of the stand foot element (2a; 2b; 2d) and thus also centrally with reference to the weights (9a-d; 9i-l).

6. Microscope stand according to one of Claims 4 or 5, **characterized in that** the nut/screw connection (7, 12a; 12f) is of self-locking design or is provided with a rotary lock.

7. Microscope stand according to one of the preceding Claims, **characterized in that** the stand foot element (2a; 2b; 2d) is designed with star-shaped support arms, and **in that** each arm has a cavity or a support step for in each case at least one weight (9a-d; 9i-l) and preferably in each case a supporting roller (14).

8. Microscope stand according to one of the preceding claims, in which both the mounting plate (10) and the stand foot element (2), and also each weight (9) respectively has a maximum weight of, for example, approximately 10-25 kg each, as defined within the scope of permissible working standards for manipulation by a person.

9. Microscope stand according to one of the preceding claims, **characterized in that** the weight(s) is/are produced from primed and/or painted grey cast iron, steel, brass or heavy metal and/or **in that** the stand foot element is produced from aluminium, in particular diecast aluminium.

10. Microscope stand according to one of Claims 1-9, **characterized in that** the weight or the weights (9a-d; 9i-l) have latching cutouts (21) which cooperate with matching catches (11) of the mounting holder (10a; 10d) the catches (11) preferably being produced from plastic, and **in that** preferably one of the latching cutouts is of cylindrical design, and the other is designed as an elongated hole.

11. Microscope stand according to one of the preceding claims, **characterized in that** the weight(s) (9) is/are supported so as to dampen vibrations with reference to the stand foot element (2) and/or with reference to the mounting holder (10).

12. Microscope stand according to Claims 1-11, **characterized in that** the stand foot element (2d; 2e) is designed to be rotationally symmetrical about the axis (8) of the column (3) and has at its centre about the axis (8) of the column a rotationally symmetrical cutout (4) for the column (3), the cutout (4) having at its end facing the floor (28) in a fashion deviating from the rotational symmetry a wedge-shaped projection or a wedge (26) which cohesively presses an inserted column tube (3) - preferably via a matching wedge surface (27) constructed there - against the opposite wall (25) of the cutout (4) by means of the dead weight of said column tube, it being possible for the column (3) to be clamped in a form-fitting fashion against the opposite wall (25) through a support wall (19) of the cutout (4) with the aid of at least one screw (24).

13. Method for mounting a microscope stand, **characterized in that** a mounting holder (10a; 10d) is provided, **in that** at least one weight (9a-d; 9i-l) is coarsely positioned on this mounting holder (10a; 10d), **in that** there is provided on the mounting holder (10a; 10d), approximately at its centre, a thread pin (12a; 12f) permanently connected to said mounting holder, and **in that** a hollow stand foot element (2a; 2d) is slipped over the mounting holder (10a; 10d) and onto the weight (9a-d; 9i-1), the thread pin (12a; 12f) penetrating a bore (6) in the stand foot element (2a; 2d), and at least two walls delimiting the cavity of the stand foot element (2a; 2d) encompassing the weight (9a-d; 9i-l), whereupon a nut (7) is screwed onto the thread pin (12a; 12f) until the mounting holder (10a; 10d) is firmly connected to the stand foot element (2a; 2d), the two walls simultaneously finely positioning the weight (9a-d; 9i-l) on the mounting holder (10a; 10d).

## Revendications

1. Pied pour microscope comportant un montant (3) et un socle (1) de pied destiné à être posé sur un sol (28) dans une pièce, au moins un corps (2) de socle de pied, lequel est conformé de façon à pouvoir être équipé d'au moins un poids (9), un dispositif de fixation (5, 6, 7, 10, 12) destiné à fixer le poids (9) au corps (2) du socle du pied, **caractérisé en ce que** le dispositif de fixation (5, 6, 7, 10, 12) comprend un dispositif de verrouillage (5, 6, 7, 12) et un support de montage (10) destiné à recevoir au moins l'un des poids (9), le dispositif de verrouillage (5, 6, 7, 12) pouvant être actionné depuis le côté du corps (2a, 2b, 2d, 2e) du socle du pied tourné vers le montant (3) et reliant en l'occurrence le support de montage (10), conjointement avec au moins un poids (9), au corps (2a, 2b, 2d, 2e) du socle du pied depuis le côté du corps (2a, 2b, 2d, 2e) du socle du pied détourné du montant (3).

2. Pied pour microscope selon la revendication 1, **caractérisé en ce qu'**au moins un poids (9a-d, 9i-l) est positionné approximativement sur le support de montage (10a, 10d) et **en ce que** le corps du socle du pied présente un corps creux comportant au moins une cavité, au moins deux parois entourant une cavité ou selon le cas au moins l'une des cavités étant conformées de telle sorte qu'elles positionnent avec précision le poids (9a-d, 9i-l) pendant l'opération de verrouillage.

3. Pied pour microscope selon la revendication 2, **caractérisé en ce qu'**au moins l'une des deux parois est conformée de façon à s'élargir de façon conique depuis le sol de son côté tourné vers le poids (9a-d, 9i-l) et vers le sol (28), en position d'utilisation, et/ou **en ce qu'**au moins l'une des parois et/ou le support de montage (10, 10b, 10d) est revêtu(e) ou sont revêtus d'une couche élastique (22a-m), de préférence dans un matériau alvéolaire.

4. Pied pour microscope selon l'une quelconque des revendications 1 à 3, **caractérisé en ce que** le dispositif de verrouillage comprend de préférence au moins un dispositif de raccordement à écrou et à vis (7, 12a, 12f), lequel traverse le corps (2a, 2b, 2d) du socle du pied de sorte que celui-ci puisse être vissé au support de montage (10a, 10b, 10d).

5. Pied pour microscope selon la revendication 4, **caractérisé en ce que** plusieurs poids (9a-d, 9i-l) sont prévus et **en ce que** le dispositif de raccordement à écrou et à vis (7, 12a, 12f) est disposé au centre du corps (2a, 2b, 2d) du socle du pied, par conséquent également de façon centrée par rapport aux poids (9a-d, 9i-l).

6. Pied pour microscope selon la revendication 4 ou 5, **caractérisé en ce que** le dispositif de raccordement à écrou et à vis (7, 12a, 12f) est conformé de façon autobloquante ou est pourvu d'une sécurité contre la rotation.

7. Pied pour microscope selon l'une quelconque des revendications précédentes, **caractérisé en ce que** le corps (2a, 2b, 2d) du socle du pied est conformé avec des bras d'appui en forme d'étoile et **en ce que** chaque bras présente une cavité ou un talon d'appui destiné à recevoir au moins un poids (9a-d, 9i-l) et de préférence une roue d'appui (14).

8. Pied pour microscope selon l'une quelconque des revendications précédentes, dont non seulement la plaque de montage (10) mais également le corps (2) du socle du pied ainsi que chaque poids (9) présentent un poids maximum de par exemple environ 10 à 25 kg défini dans le cadre des normes de travail autorisé lors du maniement par une personne.

9. Pied pour microscope selon l'une quelconque des revendications précédentes, **caractérisé en ce que** le poids ou selon le cas les poids est fabriqué ou sont fabriqués en fonte, acier, laiton ou métal lourd revêtu d'une couche d'apprêt et/ou laqué et/ou **en ce que** le corps du socle du pied est fabriqué en aluminium, en particulier en aluminium coulé sous pression.

10. Pied pour microscope selon l'une quelconque des revendications 1 à 9, **caractérisé en ce que** le poids ou selon le cas les poids (9a-d, 9i-l) possède ou possèdent des évidements (21) pour ergots, lesquels coopèrent avec des ergots (11) correspondants opposés prévus sur le support de montage (10a, 10d), les ergots (11) étant fabriqués de préférence en matière synthétique, et **en ce que** de préférence l'un des évidements pour ergots est conformé de façon cylindrique et l'autre de façon oblongue.

11. Pied pour microscope selon l'une quelconque des revendications précédentes, **caractérisé en ce que** le poids ou selon le cas les poids (9) est logé ou sont logés de façon amortie des vibrations par rapport au corps (2) du socle du pied et/ou par rapport au support de montage (10).

12. Pied pour microscope selon les revendications 1 à 11, **caractérisé en ce que** le corps (2d, 2e) du socle du pied est conformé de façon à présenter une symétrie de rotation autour de l'axe (8) du montant (3) et présente en son centre un évidement (4) à symétrie de rotation autour de l'axe (8) du montant (3) destiné au montant (3), l'évidement (4) présentant à son extrémité tournée vers le sol (28), de façon s'écartant de la symétrie de rotation, une saillie en forme de coin ou selon le cas un coin (26), laquelle ou lequel presse un tube de montant (3) inséré, de préférence par l'intermédiaire d'une surface en coin (27) correspondante opposée conformée sur celui-ci, nécessairement contre la paroi (25) opposée de l'évidement (4), sous l'effet du propre poids dudit tube de montant (3), le montant (3) pouvant être bloqué par fermeture géométrique contre la paroi (25) opposée par au moins une vis (24) insérée dans une paroi d'appui (19) de l'évidement (4).

13. Procédé de montage d'un pied pour microscope **caractérisé en ce qu'**un support de montage (10a, 10d) est préparé, **en ce qu'**au moins un poids (9a-d, 9i-l) est positionné approximativement sur ledit support de montage (10a, 10d), **en ce qu'**une tige filetée (12a, 12f) est disposée sur le support de montage (10a, 10d) à peu près en son centre de façon reliée de manière rigide à celui-ci, **en ce qu'**un corps creux (2a, 2d) de socle du pied est retourné au-dessus du support de montage (10a, 10d) et sur le poids (9a-d, 9i-l), la tige filetée (12a, 12f) traversant un trou (6) du corps (2a, 2d) du socle du pied et au moins deux parois délimitant la cavité du corps (2a, 2d) du socle du pied entourant le poids (9a-d, 9i-l), sur laquelle tige filetée (12a, 12f) un écrou (7) est vissé jusqu'à ce que le support de montage (10a, 10d) soit relié de façon rigide au corps (2a, 2d) du socle du pied, les deux parois positionnant en même temps avec précision le poids (9a-d), 9i-l) sur le support de montage (10a, 10d).
